# EUROPÄISCHE PATENTSCHRIFT

(11) **EP 3 320 759 B1**
(45) Veröffentlichungstag und Bekanntmachung des Hinweises auf die Patenterteilung: **02.01.2019**
(21) Anmeldenummer: 17754249.5
(22) Anmeldetag: 21.07.2017
(51) Int. Cl.: H05H 1/24, H05H 1/46

(54) **ELEKTRODENANORDNUNG ZUR AUSBILDUNG EINER DIELEKTRISCH BEHINDERTEN PLASMAENTLADUNG**
ELECTRODE ARRANGEMENT FOR FORMING A DIELECTRIC BARRIER PLASMA DISCHARGE
ENSEMBLE D'ÉLECTRODES POUR FORMER UNE DÉCHARGE DE PLASMA À BARRIÈRE DIÉLECTRIQUE

(30) Priorität: 30.09.2016 DE 102016118569
(43) Veröffentlichungstag der Anmeldung: 16.05.2018
(60) Teilanmeldung: 18200611.4
(73) Patentinhaber: Cinogy GmbH, 37115 Duderstadt (DE)
(72) Erfinder: WANDKE, Dirk, 37308 Heilbad Heiligenstadt (DE); TRUTWIG, Leonhard, 37115 Duderstadt (DE); HAHNL, Mirko, 37339 Berlingerode (DE); STORCK, Karl-Otto, 37115 Duderstadt (DE)
(74) Vertreter: Lins, Martina
(86) Internationale Anmeldenummer: PCT/DE2017/100612
(87) Internationale Veröffentlichungsnummer: WO 2018/059612

(56) Entgegenhaltungen:
- EP-A1- 2 953 431
- WO-A1-2010/082561
- WO-A1-2012/163876
- DE-B4-102009 060 627
- US-A1- 2013 202 496

## Beschreibung

Die Erfindung betrifft eine Elektrodenanordnung zur Ausbildung einer dielektrischen behinderten Plasmaentladung zwischen einer von einer Steuereinrichtung mit einer Wechselhochspannung gespeisten Elektrode und einer zu behandelnden Oberfläche eines elektrisch leitfähigen Körpers, die als Masseelektrode dient, wobei ein Dielektrikum die Elektrode zur zu behandelnden Oberfläche hin vollständig abdeckt und eine Anlageseite für die Oberfläche bildet.

Eine derartige flächige Elektrodenanordnung, die flexibel ausgebildet sein kann, ist durch die DE 10 2009 060 627 B4 bekannt. Die flächige Elektrode ist dabei eingebettet zwischen einer Dielektrikumunterseite und einer Dielektrikumoberseite, die sich jeweils in der Fläche über die Elektrode hinaus erstrecken und so auch den schmalen Rand der Elektrode abdecken, sodass eine Berührung der die Hochspannung führenden Elektrode ausgeschlossen ist. Ausgeschlossen ist ferner eine solche Annäherung an die Elektrode, das ein Funke überspringen könnte. Vielmehr verhindert das Dielektrikum einen galvanischen Stromfluss von der Elektrode zu der zu behandelnden Oberfläche, die als Masseelektrode dient. Die Elektrodenanordnung weist somit keine eigene Masseelektrode auf. Um die Ausbildung eines Plasmas in der Luftschicht zwischen der zu behandelnden Oberfläche und dem Dielektrikum sicherzustellen, kann bei einer glatten zu behandelnden Oberfläche die Unterseite der Elektrodenanordnung, die zu der zu behandelnden Oberfläche zeigt, mit vorstehenden Noppen ausgebildet sein, die mit ihrer Oberseite auf der zu behandelnden Oberfläche aufliegen und durchgehende Zwischenräume aufweisen, in denen sich das Plasma ausbilden kann, wenn an die Elektrode eine Wechselhochspannung angelegt wird.

Eine derartige Elektrodenanordnung lässt sich auf die zu behandelnde Oberfläche auflegen, wobei die zu behandelnde Oberfläche insbesondere auf die Haut eines menschlichen oder tierischen Körpers sein kann. Die Plasmabehandlung führt dabei zu einer porentiefen Desinfektion der Haut und verbessert die Aufnahmefähigkeit der Haut für pflegende Stoffe, die auf die behandelte Haut aufgebracht werden.

Es ist auch bekannt, dass eine Plasmabehandlung für eine Wundheilung vorteilhaft sein kann. Gemäß DE 10 2009 047 220 A1 wird in einem stiftähnlichen Gerät, das von einem Behandlungsgas durchströmt wird, ein Plasma erzeugt, das an einer düsenähnlich ausgeformten Stirnseite des Geräts austritt und auf die zu behandelnde Haut bzw. Wunde geleitet werden kann.

Die DE 10 2011 01 416 A1 offenbart eine flächige flexible Wundbehandlungseinrichtung, bei der zwei Flächenelektroden durch miteinander verwobene, isolierte elektrische Leiter gebildet sind. Zwischen den Leitern bildet sich die Hochspannung aus, die in den Luftzwischenräumen ein Plasma entstehen lassen soll. Hierzu ist erforderlich, dass die gesamte Elektrodenanordnung gasdurchlässig ist.

Die EP 2 953 431 A1 offenbart eine Elektrodenanordnung zur Ausbildung einer dielektrisch behinderten Plasmaentladung zwischen einer von einer Steuereinrichtung mit einer Wechselhochspannung gespeisten Elektrode und einer zu behandelnden Oberfläche eines elektrisch leitfähigen Körpers.

Es sind ferner Elektrodenanordnungen bekannt, mit denen ein dielektrisch behindertes Oberflächenplasma herstellbar ist. WO 2009/098662 A1 beschreibt eine derartige Anordnung, bei der eine erste flächige Elektrode und eine zweite gitterähnliche Elektrode in Höhenrichtung der Elektrodenanordnung mit Abstand zueinander in einem Dielektrikum eingebettet sind, sodass sich ein zur Ausbildung eines Plasmas geeignetes elektrisches Feld an der Dielektrikumsoberfläche ausbildet, die nahe der gitterähnlichen Elektrode angeordnet ist. An der gitterähnlichen Elektrode liegt eine Wechselhochspannung an, während die darunter befindliche flächige Elektrode an Massepotential liegt. Eine derartige Anordnung hat einen hohen Energiebedarf und einen geringen Wirkungsgrad hinsichtlich der Ausbildung des Oberflächenplasmas.

In dieser Hinsicht sind Elektrodenanordnungen vorteilhaft, deren Elektrode so ausgebildet ist, dass zwischen der flächigen Elektrode und der zu behandelnden Oberfläche als Masseelektrode ein weitgehend homogener Feldverlauf entsteht, der zu einem definierten und idealerweise gleichmäßigen Plasma führt.

Es besteht zunehmend das Bedürfnis, auch vergleichsweise große Flächen durch Auflegen einer - insbesondere flexiblen - Elektrodenanordnung der beschriebenen Art zu behandeln. Mit zunehmender Behandlungsfläche wird es allerdings schwieriger, in üblicher Technik die benötigten Feldstärken für die Ausbildung eines gleichmäßigen Plasmas zwischen der Anlagenseite des Dielektrikums und der zu behandelnden Oberfläche auszubilden. Der Erfindung liegt somit das Problem zu Grunde, eine Elektrodenanordnung der genannten Art so auszubilden, dass einerseits in effizienter Weise ein möglichst gleichmäßiges Plasma ausgebildet wird und andererseits mit einem geringeren Energieaufwand auch größere Flächen mit einer entsprechend großen Elektrodenanordnung behandelt werden können.

Zur Lösung dieses Problems ist erfindungsgemäß eine Elektrodenanordnung der eingangs erwähnten Art dadurch gekennzeichnet, dass die Elektrode aus wenigstens zwei im gleichen Abstand zur Anlagenseite nebeneinander angeordneten und durch das Dielektrikum voneinander isolierten Teilelektroden besteht und dass benachbarte Teilelektroden von der Steuereinrichtung mit bezüglich der Wellenform und der Spannungshöhe gegengleichen, sich kompensierenden Teil- Wechselhochspannungen gespeist werden.

Die erfindungsgemäße Elektrodenanordnung beruht somit auf dem bekannten Prinzip, die zu behandelnde Oberfläche des elektrisch leitfähigen Körpers als Masseelektrode zu verwenden, sodass prinzipiell für die Ausbildung eines Plasmafelds nur eine einzige Elektrode benötigt wird, die mit der zu behandelnden Oberfläche als Masseelektrode zur Ausbildung des Plasmas zusammenwirkt. Dabei entsteht über die Fläche der Elektrode weitgehend ein idealerweise homogenes elektrisches Feld, in dem die Feldlinien somit parallel zueinander verlaufen. Lediglich am Rand der Elektrode können bekannterweise gekrümmte oder schräg verlaufende Feldlinien entstehen. Bei der erfindungsgemäßen Elektrodenanordnung sind die Teilelektroden vorzugsweise und einer solchen Flächengröße ausgebildet, dass die Ausdehnung des elektrischen Feldes und idealerweise parallelen Feldlinien mehr als 50 %, vorzugsweise mehr als 65 % und weiter bevorzugt mehr als 80 % der Fläche der Teilelektrode beträgt. Die erfindungsgemäßen Elektroden sind flächig ausgedehnt und parallel zur Anlageseite des Dielektrikums positioniert. Erfindungsgemäß sind wenigstens zwei Teilelektroden vorhanden, die separat von der Steuereinrichtung mit Wechselhochspannungen versorgt werden. Die Wechselhochspannungen oszillieren dabei vorzugsweise um das Massepotential herum. Im Idealfall könnten die Wechselhochspannungen sinusförmig verlaufen. Aufgrund der in der Steuereinrichtung vorhandenen Kapazitäten und Induktivitäten können auch Schwingkreisanordnungen vorhanden sein, mit denen ein Anregungsimpuls jeweils einen hochfrequenten Oszillationsvorgang triggert.

Würden die nebeneinander angeordneten Teilelektroden phasengleich angesteuert werden, würde sich im Bereich des homogenen Feldes zwischen der betreffenden Teilelektrode und der zu behandelnden Oberfläche im Idealfall ein homogenes Plasma ausbilden. In dem Verbindungsbereich zwischen den nebeneinander angeordneten Teilelektroden würden sich die Spannungen allerdings addieren und zu unerwünschten Spannungsspitzen führen, die das gleichmäßige Plasmafeld stören würden. Darüber hinaus würden innerhalb des elektrisch leitenden Körper, an der sich zu behandelnde Oberfläche befindet, erhebliche Potentialunterschiede ausbilden, die zu unerwünschten Stromflüssen innerhalb des Körpers führen könnten. Dies kann an einem lebenden Körper zu unangenehmen und gegebenenfalls gefährlichen Erscheinungen führen.

Erfindungsgemäß ist daher vorgesehen, dass die nebeneinander befindlichen Teilelektroden mit gegengleichen Wechselhochspannungen angesteuert werden, sodass in den Randbereichen der Teilelektroden, die zwischen den benachbarten Teilelektroden liegen, ein im wesentlichen feldfreies Trenngebiet entsteht. Da dieses Trenngebiet schmal und linienförmig sein kann, werden die im Plasma gebildeten desinfizierenden Produkte, beispielsweise die sich in der Luft im Plasma ausbildenenden OH-Radikale und Ozonmoleküle, auch in dem Trenngebiet wirksam, da sie auch innerhalb ihrer sehr kurzen Lebensdauer in den Bereich der Oberflächen im Trenngebiet gelangen können.

Die erfindungsgemäße Elektrodenanordnung mit ihren wenigstens zwei Teilelektroden ist daher so ausgelegt, dass die Teilelektroden über ihre nahezu gesamte Fläche mit der zu behandelnden Oberfläche idealerweise ein im Wesentlichen homogenes Feld - und damit idealerweise ein gleichmäßiges Plasma - ausbildet und zur benachbarten Teilelektrode hin ein schmales prinzipiell feldfreies Trenngebiet entstehen lassen. Für zwei benachbarte Teilelektroden wird daher die eine Teilelektrode mit einer positiven Halbwelle der Wechselhochspannung angesteuert, während die andere Teilelektrode mit einer negativen Halbwelle angesteuert wird, sodass sich im Trenngebiet die beiden Spannungen kompensieren. In einer bevorzugten Ausführungsform sind die jeweiligen Halbwellen in Größe und Form identisch, sodass sich in dem Trenngebiet ein konstantes, sich über die Periode der Wechselhochspannung nicht änderndes mit dem Potential einstellt, das dem Massepotential der Masseelektrode entspricht. In der Praxis kann die Identität der gegengleichen Halbwellen nur angenähert vorliegen, sodass ein konstantes Summenpotential im Trenngebiet auch dann vorliegt, wenn noch eine geringe Schwankung des Summenpotentials vorliegt, die beispielsweise weniger als fünf Prozent der Scheitelspannung beträgt. Das im Idealfall gleichmäßige Plasma kann in der Praxis von geringfügigen Filamententladungen überlagert bzw. gestört sein, auch wenn angestrebt wird, solche Filamententladungen zu vermeiden.

Die Scheitelspannung der verwendeten Wechselhochspannungen kann zweckmäßigerweise zwischen ± 10 kV und ± 100 kV liegen. Die Wechselfrequenzen der Wechselhochspannungen liegen zweckmäßigerweise zwischen einigen 100 Hz und etwa 100 MHz.

Für die Anpassung an ungleichmäßige Oberflächen ist es zweckmäßig, wenn die Teilelektroden und das Dielektrikum flexibel sind. Dadurch kann die gesamte Elektrodenanordnung einer unregelmäßigen Oberfläche folgen, sodass diese im Idealfall mit einem gleichmäßigen Plasmafeld behandelt werden kann.

In an sich bekannter Weise kann die Anlageseite des Dielektrikums, die zu der zu behandelnden Oberfläche zeigt, eine Struktur, vorzugsweise in Form eines Gitters oder von Noppen, aufweisen, zwischen denen sich das Plasma ausbilden kann, wenn das Dielektrikum mit der Oberseite der Noppen oder anderer vorstehender Strukturen an der zu behandelnden Oberfläche anliegt.

Die erfindungsgemäße Elektrodenanordnung lässt sich auch als Wundauflage ausbilden, wenn das Dielektrikum aus einem wundverträglichen Material, beispielsweise geeigneten Silikonen gebildet ist oder auf die Anlageseite des Dielektrikums eine Schicht aus einem wundverträglichen Material, beispielsweise Gaze, aufgelegt wird. Die erfindungsgemäße Elektrodenanordnung eignet sich auch für die Ableitung von Wundflüssigkeit oder für die Zuleitung eines wundheilenden bzw. wundheilungsfördernden Liquids, wenn das Dielektrikum und die Teilelektroden Durchgangsöffnungen aufweisen, die sich in einer Höhenrichtung durch die Elektrodenanordnungen erstrecken und durchgehend von dem die Teilelektroden umgebenden Dielektrikum begrenzt sind.

Die erfindungsgemäße Elektrodenanordnung weist vorzugsweise bezüglich der Teilelektroden eine hohe Symmetrie auf. Hierzu ist es zweckmäßig, wenn die Teilelektroden eine gleiche Größe aufweisen, sodass die für die Ausbildung des Plasmas wirksame Fläche gleichmäßig auf die Anzahl der Teilelektroden verteilt wird.

Die Teilelektroden können aus einem flachen metallischen Material bestehen, das vorzugsweise beidseitig von einem Dielektrikum abgedeckt wird. Es ist aber auch möglich, die Elektroden durch einen leitfähigen Kunststoff zu realisieren, der sich mit dem ebenfalls durch einen Kunststoff, beispielsweise Silikon, gebildeten Dielektrikum auch formschlüssig verbinden kann. Die Elektrode kann beispielsweise aus einem Silikon mit leitfähigen Zusätzen, in Form von Metallpartikeln, Kohlestoffpartikeln o. ä., bestehen.

Die Erfindung soll im Folgenden anhand von den Zeichnungen dargestellten Ausführungsbeispielen näher erläutert werden. Es zeigen:
- Figur 1: einen Elektrodenaufbau in einer explodierten Darstellung,
- Figur 2: eine Draufsicht auf eine fertiggestellte Elektrodenanordnung gemäß Figur 1 und
- Figur 3: eine schematische Darstellung der Funktion des erfindungsgemäßen Aufbauprinzips.

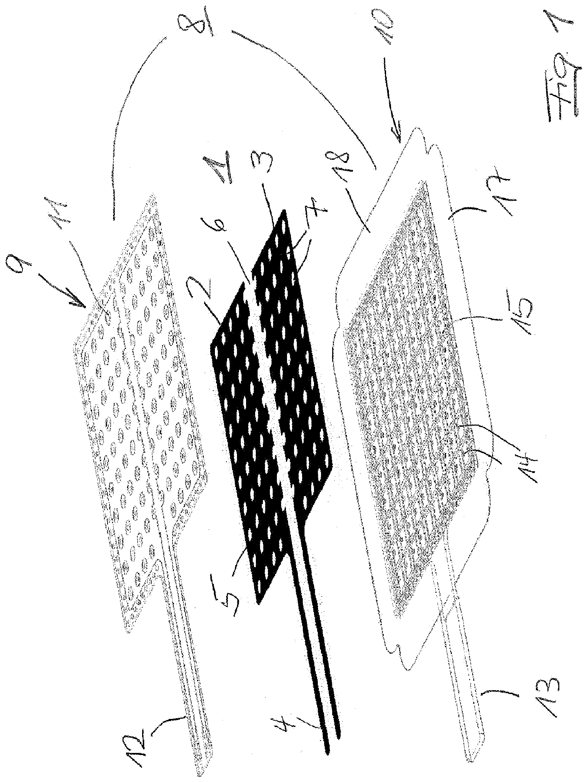

Gemäß Figur 1 ist eine Elektrode 1 durch zwei Teilelektroden 2, 3 gebildet, die nicht miteinander verbunden sind und zueinander einen definierten Abstand aufweisen. Die Teilelektroden 2, 3 bestehen jeweils aus einer schmalen flachen Zuleitung 4, die in ein flächiges Gebilde 5 übergeht. Die flächigen Gebilde 5 der beiden Teilelektroden 2, 3 bilden zusammen eine etwa quadratische Elektrodenfläche, wobei in dem dargestellten Ausführungsbeispiel ein Abstand 6 in der durch die Zuleitungen 4 definierten Längsrichtung zwischen den flächigen Gebilden 5 vorliegt.

Die flächigen Gebilde 5 der Teilelektroden 3 weisen eine Anzahl an Durchgangsöffnungen 7 auf, deren Funktion unten näher erläutert wird. Das Material der Teilelektroden kann, wie erwähnt, eine metallische Folie, ein dünnes metallisches Blech oder eine durch den Zusatz von leitenden Partikeln leitend gemachte Kunststoffschicht, insbesondere Silikonschicht, sein.

Figur 1 lässt erkennen, dass sich zu dem Abstand 6 hin in beiden flächigen Gebilden 5 der Teilelektroden 2, 3 etwa halbkreisförmige Durchgangslöcher befinden, die eine sichere Verzahnung der Teilelektroden mit einem dem Abstand 6 ausfüllenden Dielektrikum bewirken.

Die Elektrode 1 wird allseitig von einem Dielektrikum 8 abgedeckt, das in Figur 1 als aus einer oberen Dielektrikumsschicht 9 und einer unteren Dielektrikumsschicht 10 bestehend dargestellt ist. Die obere Dielektrikumsschicht 9 überragt die gemeinsame Fläche der beiden Teilelektroden 2, 3 mit ihrer Fläche allseitig und ist ebenfalls mit Durchgangsöffnungen 11 versehen, die so angeordnet sind, dass sie mit den Durchgangsöffnungen 7 der Teilelektroden 2, 3 fluchten. In dem Bereich des Abstands 6 zwischen den Teilelektroden 2, 3 ist die obere Dielektrikumsschicht massiv ausgebildet, um eine sichere elektrische Isolation zwischen den Teilelektroden 2, 3 zu bewirken. Sowohl die obere Dielektrikumsschicht 9 als auch die untere Dielektrikumsschicht 10 weisen jeweils einen Ansatz 12, 13 auf, mit dem die Zuleitungen 4 von der Umgebung abgeschirmt werden.

Die Durchgangsöffnungen 11 der oberen Dielektrikumsschicht 9 sind konzentrisch mit den Durchgangsöffnungen 7 ausgebildet, weisen jedoch einen kleineren Durchmesser auf, sodass auch im Bereich der Durchgangsöffnungen 7 eine Schicht des Dielektrikums das Material der Teilelektroden 2, 3 abschirmt. Daher ist auch über eine Flüssigkeit keine direkte elektrische Verbindung zu den Teilelektroden 2, 3 herstellbar.

Die untere Dielektrikumsschicht 10 bildet, wie die obere Dielektrikumsschicht 9, eine durchgehende zusammenhängende Schicht aus. Die obere Dielektrikumsschicht 9 kann von Durchgangsöffnungen 14 durchbrochen sein. Auch die Durchgangsöffnungen 14 sind konzentrisch mit den Durchgangsöffnungen 7 der Teilelektroden und den Durchgangsöffnungen 11 der oberen Dielektrikumsschicht ausgebildet. Auch in der unteren Dielektrikumsschicht 10 ist der Durchmesser der Durchgangsöffnungen 14 kleiner als der Durchmesser der Durchgangsöffnungen 7 der Teilelektroden 2, 3 und gleich groß wie der Durchmesser der Durchgangsöffnungen 11 der oberen Dielektrikumsschicht 9.

Auf der von den Teilelektroden 2, 3 abgewandten Seite bildet die untere Dielektrikumsschicht 10 eine Gitterstruktur 15 mit sich kreuzenden stegartigen Wänden aus, deren freie Kanten 16 eine Anlageseite definieren, mit der eine Elektrodenanordnung auf einer zu behandelnden Oberfläche aufliegen kann.

Figur 1 lässt noch erkennen, dass die durchgehende untere Dielektrikumsschicht 10 mit seitlichen Streifen 17, 18 über die Kontur der oberen Dielektrikumsschicht 9 seitlich herausragt und dadurch Ansätze bildet, mit denen die Elektrodenanordnung auf der zu behandelnden Oberfläche befestigbar ist. Hierzu können die seitlichen Streifen auf ihrer Unterseite mit einem Haftklebmittel beschichtet oder aus einem haftenden Material gebildet sein.

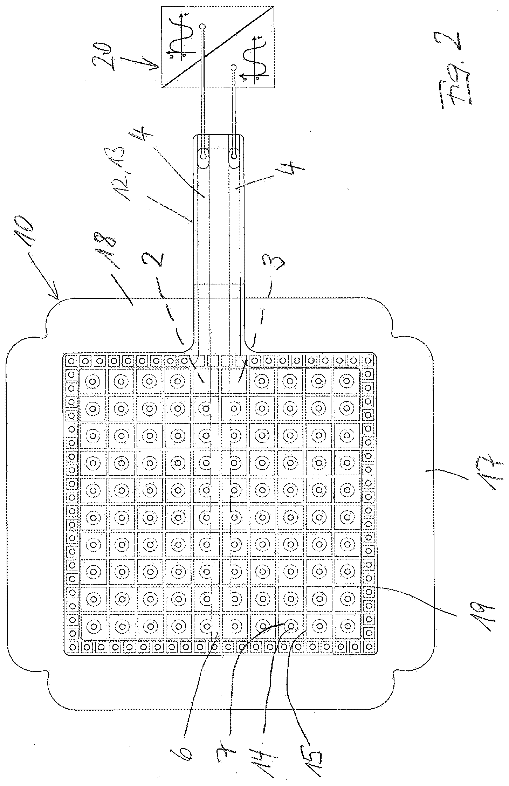

Figur 2 zeigt eine Draufsicht die Unterseite, also die Anlageseite, der Elektrodenanordnung gemäß Figur 1. Diese Darstellung lässt erkennen, dass die Gitterstruktur quadratische Kammern ausbildet, in deren Mitte sich die Durchgangsöffnungen 14 der unteren Dielektrikumsschicht befinden, die konzentrisch zu den (größeren) Durchgangsöffnungen 7 der Teilelektroden 2, 3 angeordnet sind. Auf diese Weise bilden sich durch die miteinander fluchtenden Durchgangsöffnungen 7, 14 durchgehende Kanäle, die allseits von dem Material des Dielektrikums 8 begrenzt sind und insbesondere das Material der Teilelektroden 2, 3 auch im Bereich der Durchgangsöffnungen 7 abschirmen.

Die Figur 2 lässt ferner erkennen, dass im Bereich des Abstands 6 zwischen den Teilelektroden 2, 3 das Dielektrikumsmaterial massiv ausgebildet ist. Die Gitterstruktur 15 ist außerhalb des Bereichs der Teilelektroden 2, 3 mit einer Randstruktur 19 aus rahmenförmig angeordneten kleinen Kammern verstärkt.

In Figur 2 ist angedeutet, dass die Zuleitungen 4 mittels einer schematisch dargestellten Kontaktanordnung in einer Steuereinrichtung 20 kontaktiert werden. Selbstverständlich ist dabei darauf zu achten, dass ein Berührungsschutz gegenüber den die Hochspannung führenden Zuleitungen 4 gewährleistet ist. Hierfür kann die Kontaktierung der Zuleitungen 4 beispielsweise durch Schneidkontakte erfolgen, die selbsttätig durch das Material des Dielektrikums 8 bis zu den leitenden Zuleitungen 4 einschneiden und dabei ein isolierendes Gehäuse schließen. Derartige Schneidkontaktierungen sind handelsüblich und müssen hier nicht näher erläutert werden. Schematisch ist aber angedeutet, dass die Zuleitungen mit Wechselhochspannungen versorgt werden, die hinsichtlich einer Periode so gegeneinander verschoben sind, dass sie sich in der Summe, im Idealfall zu Null, kompensieren.

Das Umschließen der Teilelektroden 2, 3 mit ihren Zuleitungen 4 mit dem Material des Dielektrikums 8 kann in üblicher Weise erfolgen. In der Anordnung gemäß Figur 1 sind die obere Dielektrikumsschicht 9 und die untere Dielektrikumsschicht 10 so ausgebildet, dass sie als thermoplastisches Material miteinander verschweißt werden oder auch lediglich miteinander verklebt werden können. Selbstverständlich ist es ebenso möglich, das gesamte Dielektrikum mit den eingelegten Teilelektroden 2, 3 in einem Gießvorgang einstückig herzustellen.

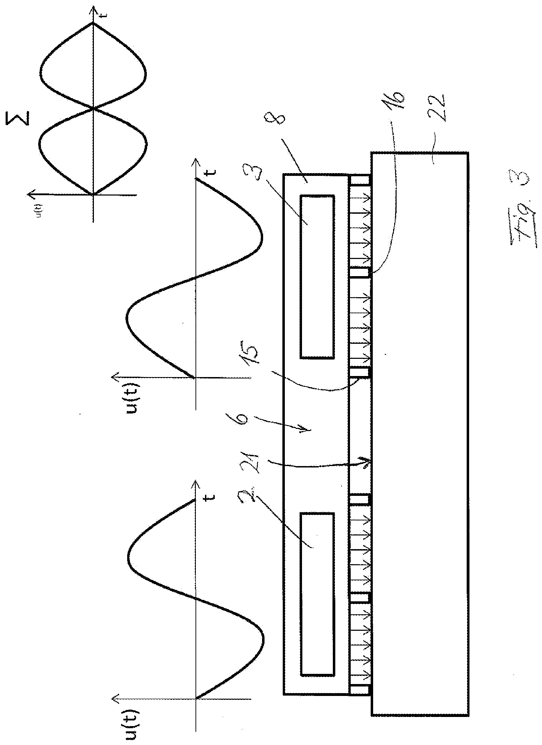

Figur 3 verdeutlicht schematisch, dass die Teilelektroden 2, 3, die in das Dielektrikum 8 eingebettet und über den Abstand 6 voneinander isoliert sind, in dem Bereich der Gitterstruktur 15, die als Abstandshalter fungiert, die Ausbildung eines Plasmas bewirken, dass dadurch homogen ist, dass das das Plasma auslösende elektrische Feld homogen zwischen den Teilelektroden 2, 3 und der Oberfläche 21 verläuft, was hier durch parallel zueinander ausgerichtete Feldlinien verdeutlicht ist. Ferner wird deutlich, dass in dem Bereich des Abstands 6 kein Plasma ausgebildet wird, weil dieser Bereich praktisch feldfrei ist. Dies ist darauf zurückzuführen, dass die beiden Teilelektroden 2, 3 mit bezüglich der Wellenform und Größe gegengleichen Wechselhochspannungen angesteuert werden, wie dies in Figur 3 schematisch oberhalb der Teilelektroden 2, 3 eingezeichnet ist. Die ebenfalls eingezeichnete Summenkurve zeigt, dass das resultierende Feld im Bereich des Abstands 6 Null ist, weil sich die beiden Wechselhochspannungen im Idealfall zu Null auslöschen. Dadurch wird verhindert, dass im Bereich zwischen den Teilelektroden 2, 3 die Plasmabildung verzerrende Felderscheinungen auftreten. Insbesondere werden Spannungsspitzen vermieden.

Die Ausbildung der Elektrode 1 mit zwei Teilelektroden 2, 3 ist bevorzugt, weil sie am einfachsten zu realisieren ist. Es ist aber für größere zu behandelnde Oberflächen denkbar, auch eine Anordnung mit beispielsweise vier Teilelektroden vorzusehen, die beispielsweise mit vier quadratischen flächigen Gebilden 5 eine gemeinsam quadratische Elektrodenfläche ausbilden. Die Ansteuerung der Teilelektroden würde dann diagonal mit gleichen Wellenformen und benachbart mit gegengleichen Wellenformen erfolgen.

Selbstverständlich sind auch andere Geometrien der Teilelektroden denkbar, beispielsweise in Form von Dreiecken, Rauten, Sechsecken oder auch Kreisflächen.

## Patentansprüche

1. Elektrodenanordnung, die eine Elektrode (1) und ein Dielektrikum (8) umfasst, zur Ausbildung einer dielektrisch behinderten Plasmaentladung zwischen der von einer Steuereinrichtung (20) mit einer Wechselhochspannung gespeisten Elektrode (1) und einer zu behandelnden Oberfläche (21) eines elektrisch leitfähigen Körpers (22), wobei das Dielektrikum (8) die Elektrode (1) zur zu behandelnden Oberfläche (21) hin vollständig abdeckt und eine Anlageseite für die Oberfläche (21) bildet; und die Elektrode (1) aus wenigstens zwei im gleichen Abstand (6) zur Anlageseite nebeneinander angeordneten und durch das Dielektrikum (8) voneinander isolierten Teilelektroden (2, 3) besteht und dass benachbarte Teilelektroden von der Steuereinrichtung mit bezüglich der Wellenform und der Spannungshöhe gegengleichen, sich kompensierenden Teil-Wechselhochspannungen gespeist werden,
**dadurch gekennzeichnet, dass** der elektrisch leitfähigen Körper als Masseelektrode dient.

2. Elektrodenanordnung nach Anspruch 1, **dadurch gekennzeichnet, dass** die Summe der benachbarten Teilelektroden (2, 3) zugeführten Teil-Wechselhochspannungen einen zeitlich konstanten Wert bildet, der dem Potential der Masseelektrode entspricht.

3. Elektrodenanordnung nach Anspruch 1 und 2, **dadurch gekennzeichnet, dass** die Teilelektroden (2, 3) und das sie abdeckende Dielektrikum (8) eine flächige Oberfläche (21) aufweisen.

4. Elektrodenanordnung nach einem der Ansprüche 1 bis 3, **dadurch gekennzeichnet, dass** die Teilelektroden (2, 3) und das Dielektrikum (8) flexibel sind.

5. Elektrodenanordnung nach einem der Ansprüche 1 bis 4, **dadurch gekennzeichnet, dass** die zur zu behandelnden Oberfläche (21) zeigende Anlageseite des Dielektrikums (8) eine Struktur aufweist, die Zwischenräume ausbildet, wenn die Elektrodenanordnung an der zu behandelnden Oberfläche (21) anliegt.

6. Elektrodenanordnung nach einem der Ansprüche 1 bis 5, **dadurch gekennzeichnet, dass** das Dielektrikum (8) und die Teilelektroden (2, 3) Durchgangsöffnungen (14) aufweisen, die sich in einer Höhenrichtung durch die Elektrodenanordnung erstrecken und durchgehend von dem die Teilelektroden (2, 3) umgebenden Dielektrikum (8) begrenzt sind.

7. Elektrodenanordnung nach einem der Ansprüche 1 bis 6, **dadurch gekennzeichnet, dass** die Teilelektroden (2, 3) eine gleiche Größe aufweisen.

## Claims

1. Electrode arrangement comprising an electrode (1) and a dielectric (8), for forming a dielectric barrier plasma discharge between the electrode (1), which is fed with a high AC voltage by a control device (20) and a surface (21) to be treated of an electrically conductive body (22), wherein the dielectric (8) completely covers the electrode (1) towards the surface (21) to be treated and forms a bearing side for the surface (21); and the electrode (1) consists of at least two partial electrodes (2, 3) which are arranged alongside one another at the same distance (6) from the bearing side and are insulated from one another by the dielectric (8) and wherein adjacent partial electrodes are fed by the control device with mutually compensating partial high AC voltages that are equal and opposite to each other with respect to waveform and voltage magnitude,
**characterized in that** the electrically conductive body serves as a ground electrode.

2. Electrode arrangement according to Claim 1, **characterized in that** the sum of the partial high AC voltages fed to adjacent partial electrodes (2, 3) forms a value which is constant over time and which corresponds to the potential of the ground electrode.

3. Electrode arrangement according to Claims 1 and 2, **characterized in that** the partial electrodes (2, 3) and the dielectric (8) covering them have a planar surface (21) .

4. Electrode arrangement according to any of Claims 1 to 3, **characterized in that** the partial electrodes (2, 3) and the dielectric (8) are flexible.

5. Electrode arrangement according to any of Claim 1 to 4, **characterized in that** the bearing side of the dielectric (8) facing the surface (21) to be treated has a structure that forms interspaces when the electrode arrangement bears against the surface (21) to be treated.

6. Electrode arrangement according to any of Claims 1 to 5, **characterized in that** the dielectric (8) and the partial electrodes (2, 3) have through openings (14) which extend through the electrode arrangement through a height direction and are delimited continuously by the dielectric (8) surrounding the partial electrodes (2, 3).

7. Electrode arrangement according to any of Claims 1 to 6, **characterized in that** the partial electrodes (2, 3) have an identical size.

## Revendications

1. Ensemble d'électrode comportant une électrode (1) et un diélectrique (8) pour engendrer une décharge de plasma à barrière diélectrique entre l'électrode (1) alimentée en haute-tension alternante par un dispositif de commande (20) et une surface à traiter (21) d'un corps électriquement conducteur (22),
dans lequel
le diélectrique (8) recouvre complètement l'électrode (1) vers la surface à traiter (21) et constitue une face d'appui pour la surface (21); et
l'électrode (1) est constituée par au moins deux électrodes partielles (2, 3) agencées l'une à côté de l'autre à la même distance (6) par rapport à la face d'appui et isolées l'une de l'autre par le diélectrique (8), et
des électrodes partielles voisines sont alimentées par le dispositif de commande en hautes-tensions alternatives partielles qui s'opposent par rapport à la forme d'onde et par rapport au niveau de tension, et qui et se compensent mutuellement,
**caractérisé en ce que** le corps électriquement conducteur sert d'électrode de masse.

2. Ensemble d'électrode selon la revendication 1, **caractérisé en ce que** la somme des hautes-tensions partielles amenées aux électrodes partielles voisines (2, 3) constitue une valeur constante dans le temps qui correspond au potentiel de l'électrode de masse.

3. Ensemble d'électrode selon la revendication 1 et 2, **caractérisé en ce que** les électrodes partielles (2, 3) et le diélectrique (8) qui les recouvre présentent une surface plane (21).

4. Ensemble d'électrode selon l'une des revendications 1 à 3, **caractérisé en ce que** les électrodes partielles (2, 3) et le diélectrique (8) sont flexibles.

5. Ensemble d'électrode selon l'une des revendications 1 à 4, **caractérisé en ce que** la face d'appui du diélectrique (8) dirigée vers la surface à traiter (21) présente une structure qui forme des intervalles lorsque l'ensemble d'électrode prend appui contre la surface à traiter (21).

6. Ensemble d'électrode selon l'une des revendications 1 à 5, **caractérisé en ce que** le diélectrique (8) et les électrodes partielles (2, 3) présentent des ouvertures traversantes (14) qui s'étendent dans une direction en hauteur à travers l'ensemble d'électrode et qui sont délimitées en continu par le diélectrique (8) entourant les électrodes partielles (2, 3).

7. Ensemble d'électrode selon l'une des revendications 1 à 6, **caractérisé en ce que** les électrodes partielles (2, 3) présentent une taille identique.
